# EUROPÄISCHE PATENTSCHRIFT

(11) **EP 1 194 123 B1**
(45) Veröffentlichungstag und Bekanntmachung des Hinweises auf die Patenterteilung: **11.04.2012**
(21) Anmeldenummer: 00947962.7
(22) Anmeldetag: 10.07.2000
(51) Int. Cl.: A61K 9/51, A61K 9/14, C08J 3/12

(54) **VERFAHREN ZUR SCHONENDEN HERSTELLUNG VON HOCHFEINEN MIKROPARTIKELN UND NANOPARTIKELN**
METHOD FOR CONTROLLED PRODUCTION OF ULTRAFINE MICROPARTICLES AND NANOPARTICLES
PROCEDE POUR LA PRODUCTION DOUCE DE MICROPARTICULES ET DE NANOPARTICULES EXTRA-FINES

(30) Priorität: 13.07.1999 DE 19932157
(43) Veröffentlichungstag der Anmeldung: 10.04.2002
(73) Patentinhaber: Abbott GmbH & Co. KG, 65205 Wiesbaden (DE)
(72) Erfinder: MÜLLER, Rainer, Helmut, D-12161 Berlin (DE); KRAUSE, Karsten, D-13357 Berlin (DE); MÄDER, Karsten, D-13187 Berlin (DE)
(74) Vertreter: UEXKÜLL & STOLBERG
(86) Internationale Anmeldenummer: PCT/EP2000/006535
(87) Internationale Veröffentlichungsnummer: WO 2001/003670

(56) Entgegenhaltungen:
- EP-A- 0 778 083
- WO-A-00/25772
- WO-A-99/61001
- DE-A- 4 440 337
- US-A- 5 091 187
- US-A- 5 510 118

## Beschreibung

Die Erfindung betrifft hochfeinene Mikropartikel und Nanopartikel und ein Verfahren zu ihrer schonenden Herstellung unter Ausschluß von Wasser bzw. Minimierung von Wasser und gegebenenfalls Ausschluß von Weichmachern und/ode bei reduzierter Temperaturbelastung.

### Hintergrund der Erfindung

Entsprechend ihrer Zusammensetzung lassen sich Mikro- und Nanopartikel in drei große Gruppen einteilen, und zwar Partikel aus:
I. reinem Arzneistoff,
II. reinem Matrixmaterial (z.B. Polymere, natürliche Makromoleküle, Lipide),
III. Wirkstoff-beladenem Matrixmaterial.

Partikelgrößen oberhalb von 10 µm sind leicht durch konventionelle Zerkleinerungstechniken zugänglich, z.B. Mahlen mit einer Mörsermühle, ggf. unter Stickstoffkühlung. Schwieriger ist es, hochfeine Partikel kleiner 10-20 µm und insbesondere Nanopartikel kleiner 1µm, insbesondere im Bereich von wenigen 100 nm, herzustellen.

Luftstrahlmahlung gibt Partikelverteilungen bis zu 25 µm (Peters, K., Nanosuspensions for the i.v. administration of poorly soluble drugs - stability during sterilization and long-term storage, 22nd Int.Symp.CRS, 1995, 2212); zusätzlich kann die Thermobelastung und die Exposition mit Sauerstoff die chemische Stabilität empfindlicher Wirkstoffe beeinträchtigen.

Naßmahlverfahren (List, P.H., Arzneiformenlehre, 3.Auflage, 1982, WVG, Stuttgart) in Wasser reduzieren bei entsprechender Kühlung zwar die Temperaturbelastung, sind jedoch für hydrolyseempfindliche Wirkstoffe ungeeignet.

Ein alternativer Herstellungsprozeß ist die Ausfällung der Partikel, z.B. zur Herstellung von Arzneistoffnanopartikeln (sog. Hydrosole) (Sucker, H., Hydrosole - eine Alternative für die parenterale Anwendung von schwer wasserlöslichen Wirkstoffen, in: Müller, R.H., Hildebrand, G.E., (Hrsg.), Pharmazeutische Technologie: Moderne Arzneiformen, 2.Auflage, 1998, WVG, Stuttgart). Nachteilig hierbei ist, daß in der Regel organische Lösungsmittel eingesetzt werden müssen (restgehalt im Produkt). Zusätzlich ist problematisch, daß der Arzneistoff zumindest in einem Lösungsmittel löslich sein muß. Gleichzeitig muß dieses Lösungsmittel noch mit einem Nicht-Lösungsmittel mischbar sein, um die Partikel durch Zugabe des Lösungsmittels zum Nicht-Lösungsmittel nach Ostwald-Mier feindispers auszufällen. Die entstehenden Partikel müssen dann noch durch geschickte Wahl der stabilisierenden Tensidmischung am Partikelwachstum während des Ausfällprozesses gehindert und für die Langzeitlagerung stabilisiert werden.

Andere Verfahren zur Herstellung von Mikro- und Nanopartikeln sind z.B. die Sprühtrocknung (Wagenaar, B.W., Müller, B.W., Piroxicam release from spray-dried biodegradable microspheres, Biomaterials 1994, 15, 49-53), solvent evaporation-Methode (Nihant, N., et al, Polylactide Microparticles Prepared by Double Emulsion/Evaporation Technique. I. Effect of Primary Emulsion Stability, Pharm. Res., 1994, 11, 1479-1484), solvent deposition und die Phasenseparation (Speiser, P.P., Nanopartikel, in: Müller, R.H., Hildebrand, G.E., (Hrsg.), Pharmazeutische Technologie: Moderne Arzneiformen, 2. Auflage, 1998, WVG, Stuttgart, 339-357). Alle beinhalten jedoch in der Regel organische Lösungsmittel, zusätzlich ist der Kontakt mit Wasser unvermeidlich (Fahr, A., Kissel, T., Mikropartikel und Implantate: Arzneiformen zur parenteralen Applikation, in: Müller, R.H., Hildebrand, G.E., (Hrsg.), Pharmazeutische Technologie: Moderne Arzneiformen, 2. Auflage, 1998, WVG, Stuttgart, 243-259).

Als alternatives Verfahren zur Herstellung von Mikro- und Nanopartikeln über Partikelzerkleinerung unter Vermeidung organischer, toxikologisch bedenklicher Lösungsmitel wurde dann die Hochdruckhomogenisation eingesetzt. Das zu zerkleinernde Polymer (Müller, B.W., Verfahren zur Herstellung von Pseudolatices und Mikro- oder Nanopartikeln und diese enthaltenden pharmazeutischen Präparaten, EP 0 605 933 B1, 1998) oder der Arzneistoff (Liversidge, G.G. Surface modified drug nanoparticles, USA-A-5 145 684, 1991; Haynes, D.H., Phospholipidcoated microcrystals: injectable formulations of water-insoluble drugs, US-A-5 091 187, 1992; Westesen, K., Solid lipid particles, particles of bioactive agents and methods for the manufacture and use thereof, International Patent Application WO 94/20072, 1994) wird in Wasser aufgeschwemmt und dann die Suspension durch den Hochdruckhomogenisator gegeben. Nachteilig ist hier, daß bei allen Verfahren die zu zerkleinernden Partikel Wasser ausgesetzt sind. Insbesondere ist beschrieben, daß bei Polymeren noch die Temperatur zu erhöhen ist und gegebenenfalls ein toxikologisch unerwünschter Weichmacher zugesetzt werden muß, z.B. 0,3 - 10 % bei Ethylcellulose (Müller, B.W., Verfahren zur Herstellung von Pseudolatices und Mikro- oder Nanopartikeln und diese enthaltenden pharmazeutischen Präparaten, EP 0 605 933 B1, 1998). Auch werden Arzneistoffe aufgeschmolzen (Westesen, K., Solid lipid particles, particles of bioactive agents and methods for the manufacture and use thereof, International Patent Application WO 94/20072, 1994), die neben der chemischen Stabilitätsbeeinträchtigung dann auch noch dazu neigen, nach der Homogenisation nicht wieder zu kristallisieren (Siekmann, B., Westesen, K., Preparation and physicochemical characterization of aqueous dispersions of coenzyme Q10 nanoparticles, Pharm. Res., 1995, 12, 201-208).

Weiterhin ist aus der US-A-5 510 118 ein Verfahren zur Herstellung von Teilchen bekannt, die im Wesentlichen aus 99,9 bis 10 Gew.-% kristallinem Wirkstoff mit geringer Wasserlöslichkeit und an der Oberfläche davon adsorbiertem, unvernetztem Oberflächenmodifizierer in einer Menge von 0,1 bis 90 Gew.-% bestehen, wobei diese Menge ausreichen soll, die Teilchengröße unter 400 nm zu halten. Bei diesem Verfahren wird ein Material mit einer Teilchengröße von etwa 100 µm bei oder unterhalb von 40 °C in einem Microfluidizer homogenisiert. Das Verfahren wird üblicherweise in Wasser als Medium der ersten Wahl durchgeführt, wobei aber auch andere Medien erwähnt sind, wie wäßrige Salzlösungen, Diestelöl, Ethanol, tert. Butanol, Hexane und Glykol.

Somit besteht generell der Bedarf, für ein schonenderes Zerkleinerungsverfahren je nach Eigenschaften des zu homogenisierenden Materials:
- den Kontakt mit Wasser zu minimieren bzw. auszuschließen
- die Verwendung toxikologisch unerwünschter organischer Lösungsmittel wie Dichlormethan auszuschließen
- die Temperaturbelastung zu minimieren bzw. zu vermeiden
- den Zusatz von toxikologisch unerwünschten Additiven wie Weichmachern zu umgehen
- die Exposition mit Sauerstoff zu minimieren bzw. auszuschließen
- Aufschmelzen zu vermeiden und die zu prozessierenden Substanzen im festen Zustand zu behalten.

Erfindungsgemäß wird ein Verfahren zur schonenden Herstellung von hochfeinen Mikro- und Nanopartikeln mit einer Partikelgröße, angegeben als mittlerer Durchmesser der Anzahlverteilung, kleiner als 1 µm bereitgestellt, das dadurch gekennzeichnet ist, daß ein Matrixmaterial in einem wasserfreien oder wasserreduzierten Dispersionsmedium, das weniger als 50 Ges.% Wasser enthält, dispergiert wird und bei niedrigen Temperaturen unter 90 °C, vorzugsweise 20 °C und insbesondere unterhalb des Gefrierpunktes von Wasser, in einem Kolben-Spalt-Homogenisator einem Hochdruckhomogenisationsprozeß unterworfen wird, der zu einer schonenden Partikelzerkleinerung führt unter Minimierung der Beeinträchtigung der chemischen Stabilität des homogenisierten Materials.

Die vorliegende Erfindung realisiert ein schonendes Zerkleinerungsverfahren durch Homogenisation, wobei je nach Eigenschaften der zu verarbeitenden Substanz ein oder mehrere dieser Parameter oder alle gleichzeitig erfüllt werden. Falls ein Parameter nicht unbedingt umgesetzt werden muß (z.B. Ausschluß von Sauerstoff ist nicht notwendig), so wird darauf aus Kostengründen verzichtet, um den Prozeß so kostengünstig wie möglich zu gestalten.

Das Zerkleinerungsprinzip der Hochdruckhomogenisation ist die Kavitation (Müller, R.H., Böhm, B.H.L., Grau, M.J., Nanosuspensions - Formulierungen für schwerlösliche Arzneistoffe mit geringer Bioverfügbarkeit: I. Herstellung und Eigenschaften, Pharm. Ind., 1999, 74-78). Wasser siedet, wenn der auf ihm lastende statische Druck (z.B. Luftdruck) gleich oder kleiner als der Dampfdruck wird. Im Hochdruckhomogenisator strömt Flüssigkeit mit sehr hoher Geschwindigkeit, so daß der statische Druck unterhalb des Dampfdruckes von Wasser sinkt, dieses in den gasförmigen Zustand übergeht und Gasblasen bildet. Beim Kollabieren der Dampfblasen (z.B. beim Austritt aus dem Homogenisationsspalt) kommt es durch diese Implosion zu starken Schockwellen, die zur Partikelzerkleinerung führen. Die Zerkleinerung von Substanzen durch Hochdruckhomogensiation erfolgte daher bisher in Wasser und nicht in Flüssigkeiten mit einem geringeren Dampfdruck. Es wird sogar die Hochdruckhomogenisation bei erhöhter Temperatur (deutlich oberhalb Raumtemperatur, z.B. bei 60-90°C) empfohlen, da dann die Differenz zwischen statischem Druck (z.B. im Homogenisationsspalt) und Dampfdruck des Wassers leichter überwunden werden kann. Insbesondere wurde Homogenisation bei tieferen Temperaturen nicht durchgeführt, da dann aufgrund des bei niederen Temperaturen kleineren Dampfdruckes des Wassers die Differenz zwischen statischem Druck und Dampfdruck zunimmt und keine Kavitation auftritt. Insbesondere beim Zerkleinern von Polymeren wird sogar Temperaturerhöhung als nicht ausreichend für eine effektive Zerkleinerung beschrieben, es müssen Weichmacher den Polymeren zugesetzt werden (Müller, B.W., Verfahren zur Herstellung von Pseudolatices und Mikro- oder Nanopartikeln und diese enthaltenden pharmazeutischen Präparaten, EP 0 605 933 B1, 1998).

Im Gegensatz zur Verwendung von Wasser werden in der Erfindung nichtwäßrige Flüssigkeiten, insbesondere auch mit niedrigerem Dampfdruck (flüssige Polyethylenglykole, wasserfreies Glycerin), im Homogensiationsverfahren eingesetzt. Überraschenderweise zeigte sich, daß auch damit hochfeine Mikropartikel und Nanopartikel hergestellt werden konnten (Beispiele 2,3). Im Vergleich zu Partikeln, die in Wasser homogenisiert wurden, ergaben sich vernachlässigbare Unterschiede. Homogenisation in wasserfreien Medien wurde für reine Wirkstoffe (z.B. Arzneistoffe, kosmetische Wirkstoffe etc.), synthetische Polymere und natürliche Makromoleküle sowie für Wirkstoff-beladene Polymere durchgeführt.

In Abhängigkeit vom Grad der Hydrolyseempfindlichkeit von Wirkstoffen können geringe Anteile von Wasser im Dispersionsmedium toleriert werden. So wurden dem Dispersionsmedium Anteile von Wasser zugesetzt, um dadurch die Einheitlichkeit der Partikeldispersion zu verbessern. Der mittlere Durchmesser der Partikeldispersion zeigt kaum Veränderung im Vergleich zu wasserfreiem Dispersionsmedium. Es sinkt jedoch leicht der Durchmesser 95%, der ein Maß für die Anwesenheit weniger größerer Partikel neben der Hauptpopulation der Teilchen ist. Unabhängig davon sind gewisse Wasseranteile oft in der Weiterverarbeitung der Partikeldispersion erwünscht (z.B. in PEG 400 bei Abfüllung in Weichgelatinekapseln sollte das PEG einen gewissen Feuchtmacheranteil enthalten, damit der Gelatine-Kapselwand selbst kein Wasser entzogen wird und dadurch die Kapsel spröde wird). Voraussetzung hierfür ist jedoch zumindest eine geringe Löslichkeit von Wasser im Dispersionsmedium bzw. Mischbarkeit. Zugesetzte Wasseranteile waren z.B. 1%, 5% und 10%. Überraschenderweise hatten diese Wasseranteile - entgegen den theoretischen Überlegungen - keinen die Zerkleinerung erhöhenden Einfluß (wenig Änderung im Durchmesser 50%).

Es wurden auch höhere Wasseranteile eingesetzt (maximal eingesetzte Wassermengen waren <50%), wobei sich die Partikelgröße im Vergleich zum wasserfreien Medium unwesentlich bzw. nicht verkleinerte. Für die meisten Produkte sind derartige minimale Unterschiede für die Produktqualität belanglos. Für Suspensionen zur intravenösen Injektion ist es zur Vermeidung der Kapillarblockade belanglos ob der mittlere Durchmesser bei 0,6 6 µm oder 0,7 µm liegt, solange man zur Vermeidung von Kapillarblockade (Embolie) deutlich unterhalb der kleinsten Größe von Kapillaren von 5-6 µm bleibt. Diese Ergebnisse bestätigen, daß eine äußere Wasserphase zur Erzielung eines Produktes mit ausreichender Feinheit nicht notwendig ist.

Der Anteil an Mikropartikeln mit einer Größe deutlich oberhalb des mittleren Durchmessers 50% ist eine Funktion der Anzahl der Homogenisationszyklen. Er sinkt (d.h. der D95% bzw. D90% als Maß für diesen Anteil wird kleiner) mit steigender Zahl der Zyklen . Zur Reduzierung des Anteils an Mikropartikeln - z.B. im Hinblick auf i.v. Applikation - kann generell die Zahl der Zyklen erhöht werden, so daß auch hierfür ein Wasserzusatz zum Dispersionsmedium nicht erforderlich ist.

Ein die Stabilität von Wirkstoffen noch nicht beeinträchtigender Wasserzusatz ist auch dann sinnvoll, wenn in diesem Wasser Substanzen oder Polymere gelöst werden, die im nichtwäßrigen Lösungsmittel nicht oder nicht ausreichend löslich sind, aber für die Endformulierung erwünscht sind. Beispiele sind Hydroxypropylmethylcellulose (HPMC) als Gerüstbildner oder PEG 6000 als Formtrennmittel, wenn die Mikro- oder Nanopartikeldispersion in eine trockene Formulierung wie Tablette oder Pellet überführt werden soll. Sinnvoll sind ebenfalls Gelbildner für die nachträgliche Herstellung von Gelen, z.B. Miglyolgel (Lösung von Aerosil mit geringem Wasseranteil zur Förderung der Gelbildung im Öl über Hydroxylgruppen des Wassers).

Zur Untersuchung des Einflusses eines Weichmachers wurde vergleichbar zu Müller, B.W., Verfahren zur Herstellung von Pseudolatices und Mikro- oder Nanopartikeln und diese enthaltenden pharmazeutischen Präparaten, EP 0 605 933 B1, 1998 Ethylcellulose unter Zusatz von 1,74 % (m/m bezogen auf das Polymer) Weichmacher bei erhöhter Temperatur homogenisiert und verglichen mit einer ohne Weichmacherzusatz hergestellten Mikropartikel-Suspension. Die Unterschiede in der Teilchengrößen waren gering bzw. die Weichmacher-freie Dispersion zeigte sogar überraschenderweise geringere Partikelgrößen, so daß auf toxikologisch unerwünschte Weichmacher - entgegen den Erwartungen aufgrund der Literatur - verzichtet werden kann.

Für Polymere wie Ethylcellulose (Müller, B.W., Verfahren zur Herstellung von Pseudolatices und Mikro- oder Nanopartikeln und diese enthaltenden pharmazeutischen Präparaten, EP 0 605 933 B1, 1998) soll Homogenisation bei höheren Temperaturen zu kleineren Teilchen führen. Dies basiert auf den theoretischen Überlegungen, daß die Differenz zwischen statischem Druck im Homögenisator und dem Dampfdruck des Dispersionsmediums geringer ist und man sich dem Erweichungspunkt von Polymeren annähert. Ethylcellulose wurde daher bei verschiedenen Temperaturen homogenisiert und die Teilchengrößen verglichen. Die Unterschiede waren minimal und für die Produktqualität in der Regel nicht relevant. Somit kann für diese Substanzen ohne Verlust in der produktrelevanten Qualität bzgl. Partikelgröße anstatt bei 85°C auch bei 40-60° C oder leicht oberhalb bzw. bei Raumtemperatur (20° C) gearbeitet werden.

Bei der Hochdruckhomogenisation kommt es zur Dissipation von Strömungsenergie in Wärme (Jahnke, S., Theorie der Hochdruckhomogenisation, Workshop Dispergiertechnik, 4th Expert Meeting, cdc 1999), das Produkt erwärmt sich (z.B. pro Zyklus um ca. 10-20°C beim LAB 40, APV Deutschland GmbH, Lübeck, Germany). Bei sehr temperaturempfindlichen Substanzen sollte diese Wärme nicht erst im Produktcontainer dem Produkt entzogen werden sondern vorzugsweise bereits im Homogenisationsturm während des Zerkleinerungsprozesses. Die Prozeßführung erfolgt in diesen Fällen bei abgesenkter Temperatur, d.h. unter Kühlung bei 4°C oder auch deutlich unter 0°C, z.B. bei -20° oder -50°C, was nur unter Vermeidung von Wasser als reiner äußerer Phase möglich ist. Entgegen den theoretischen Überlegungen (noch niedrigerer Dampfdruck von Wasser bei diesen tiefen Temperaturen) war die Hochdruckhomogenisation überraschenderweise ausreichend effektiv zur Herstellung von hochfeinen Partikeldispersionen. Weitere Maßnahmen sind Entgasung des Dispersionsmediums (z.B. im Vakuum oder durch Erhitzen) und zusätzlich Schutzbegasung (z.B. mit Stickstoff).

### Detaillierte Beschreibung der Erfindung

Die in hochfeine Mikropartikel oder Nanopartikel zu überführende Substanz (z.B. Wirkstoffe, Polymere oder Wikstoff-beladene Polymere) wird als Pulver unter Rühren in einem flüssigen Medium (Dispersionsmedium) zur Herstellung einer Prä-Suspension dispergiert. Dispergierung kann mit Rührern unterschiedlicher Bauart erfolgen, z.B. Propellerrührer, Rotor-Stator-Rührer (Ultra-Turrax), Zahnscheiben. Alternativ kann die gepulverte Substanz auch angerieben werden, z.B. in einer Mörsermühle. Der Substanz in der Mörsermühle wird sukzessive Dispersionsmedium unter Rühren zugesetzt.

Als Dispersionsmedien können alle Flüssigkeiten außer Wasser mit ausreichend niedriger Viskosität eingesetzt werden, z.B.

Polyole wie z.B. Glycerin, Polyethylenglykole (PEG) (z.B. PEG 400 und PEG 600), Polyether- und Polyesterpolyole, Glykole wie z.B. Propylenglykol, Ethylenglykol,

Öle wie z.B. mittelkettige Triglyceride (MCT) (z.B. Miglyole), langkettige Triglyceride (LCT) wie z.B. Isopropylmyristat, pflanzliche Öle wie Avocadoöl, Baumwollsamenöl, Distelöl, Erdnußöl, Jojobaöl, Kokosnußöl, Leinöl, Nußöl, Olivenöl, Palmkernöl, Sesamöl, Sojabohnenöl, Rizinusöl, Weizenkeimöl, tierische Öle wie Lebertran, Heilbuttleberöl, Rinderklauenöl,

flüssige Kohlenwasserstoffe wie z.B. dünnflüssiges Paraffin, dickflüssiges Paraffin und Hexan

Alkohole wie Methanol, Ethanol, 1-Propanol, Isopropanol, n-Butanol, 2-Butanol, Pentanol, Hexanol, Octanol, Decanol, Allylalkohol, Propargylalkohol.

Falls für das Endprodukt wünschenswert, kann dem Dispersionsmedium ein Wasseranteil zugemischt werden (z.B. Wasserzusatz zu PEG 400 im Hinblick auf eine spätere Befüllung von Weichgelatinekapseln). Die Wasseranteile bewegen sich in der Regel im Bereich von 1 bis 10%, es können aber auch höhere Anteile verwendet werden. Limitierender Faktor ist hierbei die chemische Stabilität der zu homogenisierenden Substanz. Höhere Anteile von Wasser haben zwar keinen oder wenig Effekt auf den mittleren Durchmesser der hergestellten Partikeldispersion, es wird jedoch der Anteil an größeren Partikeln zusätzlich minimiert. Der Durchmesser 95% sinkt in der Regel leicht. Für viele Produkte ist dies von keiner Relevanz. Interessant ist es jedoch bei der Herstellung von Nanopartikeldispersionen zur intravenösen Injektion. Verbleiben zu viele Partikel größer als 5 µm im Produkt, so kann dies zu Kapillarblockade führen.

Im Wasseranteil können auch Substanzen wie HPMC, PEG 6000 oder Aerosil aufgelöst werden, wenn dies für die angestrebete Endformulierung wünschenswert ist, zu der die Mikro- und Nanopartikeldispersionen verarbeitet werden sollen. Insbesondere sind diese bezüglich der Tablettenherstellung z.B. Calciumphosphate, Lactose, Stärke und ihre Derivate wie Stärkehydrolysate, Cellulosen, Cellulosederivate, Polyethylenglykole, Polyvinylpyrrolidon (PVP), Hexite, Glucose; bezüglich der Salbenherstellung kommen Substanzen wie Bentonit, Aerosil, Celluloseether, Celluloseester, Alginate, Pectinate, Tragant, Polyvinylalkohol, Polyethylenglykole, Gummi arabicum, Polyacrylate, Paraffin, Polymethacrylate, Vaselin, Plastibase in Betracht; und bezüglich der Verarbeitung in Kapseln sind z.B. Polyethylenglykole, Paraffin, flüssige Triglyceride (pflanzlich und tierisch) von Bedeutung.

Zur Stabilisierung der Suspension und der aus dieser hergestellten Mikro- und Nanopartikel können dem Dispersionsmedium stabilisierende Substanzen zugesetzt werden. Beispiele dafür sind:
1. sterisch stabilisierende Substanzen wie Poloxamere und Poloxamine (Polyoxyethylen-Polyoxypropylen-Block-Copolymere), ethoxylierte Sorbitanfettsäure-Ester, besonders Polysorbate (z.B. Polysorbat 80 bzw. Tween 80®), ethoxylierte Mono- und Diglyceride, ethoxylierte Lipide, ethoxylierte Fettalkohole oder Fettsäuren, und Ester und Ether von Zuckern oder von Zuckeralkoholen mit Fettsäuren oder Fettalkoholen (z.B. Saccharose-stearat, Saccharose-distearat, Saccharose-laurat, Saccharose-octanoat, Saccharosepalmitat, Saccharose-myristat).
2. geladene ionische Stabilisatoren so wie Diacetylphosphate, Phosphatidylglycerin, Lecithine unterschiedlicher Herkunft (z.B. Eilecithin oder Sojalecithin), chemisch modifizierte Lecithine (z.B. hydrierte Lecithine), genauso wie Phospholipide und Sphingolipide, Mischung von Lecithinen mit Phospholipiden, Sterolen (z.B. Cholesterol und Cholesterol-Derivate, genauso wie Stigmasterin) und ebenfalls gesättigte und ungesättigte Fettsäuren, Natriumcholat, Natriumglycocholat, Natriumtaurocholat, Natriumdeoxycholat oder ihrer Mischungen, Aminosäuren oder Anti-Flokkulantien, wie z.B. Natriumcitrat, Natriumpyrophosphat, Natriumsorbat [Lucks, J.S. et al. Int. J. Pharm., 1990, 58, 229 - 235]. Zwitterionische Tenside wie z.B. (3-[(3-cholamidopropyl)-dimethylammonio]-2-hydroxy-1-propanesulfonate) [CHAPSO], (3-[(3-cholamido-propyl)-dimethylammonio]-1-propanesulfonate) [CHAPS] und N-dodecyl-N,N-dimethyl-3-ammonio-lpropansulfonat.Kationische Tenside, insbesondere als Konservierungsmittel eingesetzte Verbindungen, wie z.B. Benzyldimethylhexadecylammoniumchlorid, Methylbenzethoniumchlorid, Benzalkonium-chlorid, Cetylpyridiniumchlorid.

Die im Prozeß zur Herstellung von hochfeinen Mikropartikeln und Nanopartikeln einsetzbaren Substanzen sind
1. Reinsubstanzen (z.B. Wirkstoffe im pharmazeutischen und kosmetischen Bereich)
2. Polymere
3. Wirkstoff-beladene Polymere

Die Reinsubstanzen beschränken sich nicht nur auf z.B. Wirkstoffe im pharmazeutischen und kosmetischen Bereich sondern stammen aus sehr unterschiedlichen Bereichen (z.B. Agrarwirtschaft, Nahrungsmittel). Im Agrarbereich sind eine Reihe von Pestiziden instabil in Wasser. Sie werden daher in der Ölphase einer Emulsion gelöst und diese hoch konzentriert hergestellt, um den Wasseranteil zu minimieren. Trotzdem ist die Lagerfähigkeit beschränkt. Mit vorliegendem Verfahren können chemisch labile Pestizide in einem wasserfreien Verfahren schonend in feine Nanopartikeldispersionen überführt werden, die dann auf Pflanzen aufgebracht werden können. Hier bietet sich bevorzugt die Homogenisation in mit Wasser mischbaren Dispersionsmedien an, z.B. PEG 400. Vor dem Versprühen mischt man die in PEG dispergierten Nanopartikel Wasser zu und versprüht mit konventionellen Sprühgeräten.

Im Nahrungsmittelbereich kommen beispielsweise Geschmacksverstärker als Wirkstoffe in Frage.

Ferner sind auch Holzschutz- oder Pflegemittel als Wirkstoffe von Interesse.

Im pharmazeutischen Bereich sind vor allem Wirkstoffe interessant, die eine zu geringe Bioverfügbarkeit haben und/oder in Wasser chemisch instabil sind. Ein klassisches Beispiel ist Cyclosporin, das bisher als Mikroemulsion (kritische Lösung) auf dem Markt ist. Der Nachteil der Mikroemulsion ist der initial hohe Plasmapeak, der die Nephrotoxizität begründet. Durch Überführung in eine Nanosuspension erhöht man die Lösungsgeschwindigkeit und damit die Bioverfügbarkeit im Vergleich zum gepulverten Wirkstoff, gleichzeitig vermeidet man die schnelle Wirkstoffdiffusion aus einer Lösung. Ein anderes Beispiel ist die HIV-wirksame Substanz Azodicarbonamid (ADA). Überführung von ADA in Nanopartikel unter Verwendung von Wasser als Dispersionsmedium führt zu einer schaumigen Dispersion. Der gebildete Mikroschaum bleibt über mehrere Wochen stabil, das schaumige Produkt ist so nicht weiterverarbeitbar.

In dieser Erfindung zu verarbeitende Arzneistoffe sind z.B. aus den therapeutischen Gruppen:
Analgetika/Antirheumatika
   BTM Basen wie Morphin, Codein, Piritamid, Fentanyl und Fentanylderivate, Levomethadon, Tramadol, Diclofenac, Ibuprofen, Indometacin, Naproxen, Piroxicam, Penicillamin
Antiallergika
   Pheniramin, Dimetinden, Terfenadin, Astemizol, Loratidin, Doxylamin, Meclozin, Bamipin, Clemastin
Antibiotika/Chemotherapeutika
   hiervon: Polypeptidantibiotika wie Colistin, Polymyxin B,
   Teicplanin, Vancomycin; Malariamittel wie Chinin, Halofantrin, Mefloquin, Chlaroquin, Virustatika wie Ganciclovir, Foscarnet, Zidovudin, Aciclovir und andere wie Dapson, Fosfomycin, Fusafungin, Trimetoprim
Antiepileptika
   Phenytoin, Mesuximid, Ethosuximid, Primidon, Phenobarbital, Valproinsäure, Carbamazepin, Clonazepam
Antimykotika
   a) intern:
      Nystatin, Natamycin, Amphotericin B, Flucytosin, Miconazol, Fluconazol, Itraconazol
   b) extern außerdem:
      Clotrimazol, Econazol, Tioconazol, Fenticonazol, Bifonazol, Oxiconazol, Ketoconazol, Isoconazol, Tolnaftat
Corticoide (Interna)
   Aldosteron, Fludrocortison, Betametason, Dexametason, Triamcinolon, Fluocortolon, Hydroxycortison, Prednisolon, Prednyliden, Cloprednol, Methylpredinsolon
Dermatika
   a) Antibiotika:
      Tetracyclin, Erythromycin, Neomycin, Gentamycin, Clindamycin, Framycetin, Tyrothricin, Chlortetracyclin, Mipirocin, Fusidinsäure
   b) Virustatika wie oben, außerdem:
      Podophyllotoxin, Vidarabin, Tromantadin
   c) Corticoide wie oben, außerdem:
      Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid
Diagnostika
   a) radioaktive Isotope wie Te99m, Inlll oder I131, kovalent gebunden an Lipide oder Lipoide oder andere Moleküle oder in Komplexen
   b) hochsubstituiertre iodhaltige Verbindungen wie z.B. Lipide
Hämostyptika/Antihämorrhagika
   Blutgerinnungsfaktoren VIII, IX
Hypnotika, Sedativa
   Cyclobarbital, Pentobarbital, Phenobarbital, Methaqualon (BTM), Benzodiazepine (Flurazepam, Midazolam, Nitrazepam, Lormetazepam, Flunitrazepam, Triazolam, Brotizolam, Temazepam, Loprazolam)
Hypophysen-, Hypothalamushormone, regulatorische Peptide und ihre Hemmstoffe
   Corticotrophin, Tetracosactid, Choriongonadotropin, Urofollitropin, Urogonadotropin, Somatropin, Metergolin, Bromocriptin, Terlipressin, Desmopressin, Oxytocin, Argipressin, Ornipressin, Leuprorelin, Triptorelin, Gonadorelin, Buserelin, Nafarelin, Goselerin, Somatostatin
Immuntherapeutika und Zytokine
   Dimepranol-4-acetatamidobenzoat, Thymopentin, α-Interferon, ß-Interferon, γ-Interferon, Filgrastim, Interleukine, Azathioprin, Ciclosporine
Lokalanaesthetika
   intern:
   Butanilicain, Mepivacain, Bupivacain, Etidocain, Lidocain, Articain, Prilocain,
   extern außerdem:
      Propipocain, Oxybuprocain, Tetracain, Benzocain
Migränemittel
   Proxibarbal, Lisurid, Methysergid, Dihydroergotamin, Clonidin, Ergotamin, Pizotifen
Narkosemittel
   Methohexital; Propofol, Etomidat, Ketamin, Alfentanil, Thiopental, Droperidol, Fentanyl
Nebenschilddrüsenhormone, Calciumstoffwechselregulatoren
   Dihydrotachysterol, Calcitonin, Clodronsäure, Etidronsäure
Opthalmika
   Atropin, Cyclodrin, Cyclopentolat, Homatropin, Tropicamid, Scopolamin, Pholedrin, Edoxudin, Idouridin, Tromantadin, Aciclovir, Acetazolamid, Diclofenamid, Carteolol, Timolol, Metipranolol, Betaxolol, Pindolol, Befunolol, Bupranolol, Levobununol, Carbachol, Pilocarpin, Clonidin, Neostimgin
Psychopharmaka
   Benzodiazepine (Lorazepam, Diazepam), Clomethiazol
Schilddrüsentherapeutika
   1-Thyroxin, Carbimazol, Thiamazol, Propylthiouracil
Sera, Immunglobuline, Impfstoffe
   a) Immunglobuline allgemein und spezifisch wie Hepatitis-Typen, Röteln, Cytomegalie, Tollwut, FSME, Varicella-Zoster, Tetanus, Rhesusfaktoren
   b) Immunsera wie Botulismus-Antitoxin, Diphterie, Gasbrand, Schlangengift, Skorpiongift
   c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus Tollwut, Typhus
Sexualhormone und ihre Hemmstoffe
   Anabolika, Androgene, Antiandrogene, Gestagene, Estrogene, Antiestrogene (Tamoxifen etc.)
Zystostatika und Meta-stasenhemmer
   a) Alkylantien wie Nimustin, Melphalan, Carmustin, Lomustin, Cyclophosphamid, Ifosfamid, Trofosfamid, Chlorambucil, Busulfan, Treosulfan, Prednimustin, Thiotepa
   b) Antimetabolite wie Cytarabin, Fluorouracil, Methotrexat, Mercaptopurin, Tioguanin
   c) Alkaloide wie Vinblastin, Vincristin, Vindesin
   d) Antibiotika wie Aclarubicin, Bleomycin, Dactinomycin, Daunorubicin, Doxorubicin, Epirubicin, Idarubicin, Mitomycin, Plicamycin
   e) Komplexe von Nebengruppenelementen (z.B. Ti, Zr, V, Nb, Ta, Mo, W, Ru, Pt) wie Carboplatin, Cisplatin und Metallocenverbindungen wie Titanocendichlorid
   f) Amsacrin, Dacarbazin, Estramustin, Etoposid, Hydroxycarbamid, Mitoxanthron, Procarbazin, Temiposid
   g) Alkylamidophospholipide (beschrieben in J.M. Zeidler, F. Emling, W. Zimmermann und H.J. Roth, Archiv der Pharmazie, 324 (1991), 687)
   h) Etherlipide wie Hexadecylphosphocholin, Ilmofosin und Analoga, beschrieben in R. Zeisig, D. Arndt und H. Brachwitz, Pharmazie 45 (1990), 809-818.
   i) Taxane wie z.B. Paclitaxel

Peptid- und Proteinwirkstoffe, insbesondere auch rekombinante Peptide und Proteine, wie z.B. Cyclosporin, LH-RH-Analoga, Follikel-stimulierendes Horman (FSH), Gonadotropin Releasing Hormon Antagonist (GnRHA), Humanes Choriogonadotropin (hCG), Wachstumshormon-Releasing Faktor (GRF), Humanes Wachstumshormon (hGH), Interferon-beta la, Humanes Tumornekrosefaktor-bindendes Protein (hTBP), Humanes Interleukin-6 (hIL6), Lymphozytenaktivierungsgen 3, Typ 1 Interferon Rezeptor

Generell können allgemein Wirkstoffe aus folgenden chemischen Gruppen verwendet werden.
- hydroxylierte Kohlenwasserstoffe
- Carbonylverbindungen wie Ketone (z.B. Haloperidol), Monosaccharide, Disaccharide und Aminozucker
- Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide (z.B. Ciclosporin), Polypeptide, ß-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
- Kohlensäurederivate wie Urethane und Thiourethane, Harnstoff und Harnstoffderivate, Guanidinderivate, Hydantoine, Barbitursäurederivate und Thiobarbitursäurederivate
- Nitroverbindungen wie aromatische Nitroverbindungen und heteroaromatische Nitroverbindungen
- Amine wie aliphatische Amine, Aminoglykoside, Phenylalkylamine, Ephedrinderivate, Hydroxyphenylethanolamine, Adrenalinderivate, Amfetaminderivate, aromatische Amine und Derivate, quartäre Ammmoniumverbindungen
- schwefelhaltige Verbindungen wie Thiole und Disulfane
- Sulfone, Sulfonsäureester und Sulfonsäureamide
- Polycarbocyclen wie Tetracycline, Steroide mit aromatischem Ring A, Steroide mit alpha, beta-ungesättigter Carbonylfunktion im Ring A und alpha Ketol-Gruppe (oder Methylketo-Gruppe) am C 17, Steroide mit einem Butenolid-Ring am C 17, Steroide mit einem Pentadienolid-Ring am C 17 und Seco-Steroide
- 0-haltige Heterocyclen wie Chromanderivate
   (z.B. Cromoglicinsäure)
- N-haltige Heterocyclen wie Pyrazolderivate
   (z.B. Propyphenazon, Phenylbutazon)
- Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbanderivate, Pyrrolidinderivate, Purinderivate (z.B. Allopurinol), Xanthinderivate, 8-Hydroxychinolinderivate, Amino-hydroxy-alkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin)
   und Dibenzazepinderivate (z.B. Trimipramin)
- S-haltige Heterocyclen wie Thioxanthenderivate
   (z.B Chlorprothixen)
- N,0- und N,S-haltige Heterocyclen wie monocyclische N,0-haltige Heterocyclen, monocyclische N,S-haltige Heterocyclen, Thiadiazinderivate, bicyclische N,S-haltige Heterocyclen, Benzothiadiazinderivate, tricyclische N,S-haltige Heterocyclen und Phenothiazinderivate
- 0,P,N-haltige-Heterocyclen (z.B. Cyclophosphamid)

An Polymeren können synthetische, halbsynthetische sowie natürliche eingesetzt werden. Insbesondere kommen in Frage z.B.
Cellulosederivate wie Ethylcellulose, Methylcellulose, Hydroxyethylcellulose, Hydroxypropylcellulose, Natriumcarboxymethyl-cellulose, Methylhydroxypropylcellulose, Hydroxypropylmethylcelluloseacetatsuccinat, Carboxymethylcellulose, Celluloseacetatphthalat, Methylhydroxyethylcellulose,
natürliche Polymere wie Alginate, Albumin, insbesondere Serumalbumin, Humanalbumin und-bovines Albumin, Schellack, Wachse, Bienenwachs, Glanz-wachse, Kollagen, Kasein, Fibrin, Bentonit, Tragant, Xanthane, Polysaccharide wie Chitin, Dextrane, Hyaluronsäure
synthetische Polymere wie Polyacrylate, Polymethacrylate, Polyvinylderivate Polyesterpolymere wie Polylactide, Polyglycolide und ihre Ko-Polymere, Polyanhydride, Polyphosphorester, Blockpolymere aus Polyethylenglycol und Polyestern, Polyhydroxybuttersäure, Polycyanoacrylate, Polycarbonate, Polycaprolacton.

In die Polymere können auch bereits vor der Homogenisation Wirkstoffe eingeschlossen sein, z.B. aus den oben genannten therapeutischen Gruppen und / oder chemischen Gruppen. Die Wirkstoffe können in den Polymeren z.B. gelöst, dispergiert, lösungsvermittelt oder auf andere Weise eingeschlossen sein. Die Prä-Suspension wird dann -z-B. in einem : Hochdruckhomogenisatoren vom Typ des Kolben-Spalt-Homogenisators (z.B.) APV Gaulin Systeme, French Press, Avestin) weitervevanbeitet.

Die hergestellte Prä-Suspension wird bei ca. 100 bar bis ca. 2000 bar unter Anwendung von einem, mehreren oder vielen Zyklen homogenisiert. Die anzuwendenden Drücke im Hochdruckhomogenisator und die Zahl der Zyklen sind eine Funktion von der gewünschten Feinheit der Partikel. In der Regel erfordert die Herstellung von Nanopartikeln höhere Drücke (z.B. 1000 bar oder darüber) und eine höhere Anzahl an Zyklen. Die Zahl der Zyklen hängt ebenfalls von der Leistungsfähigkeit des Homogenisators ab (z.B. 4 - 20 Zyklen bei APV Gaulin Maschinen, teilweise bis zu 50 bzw. mehreren hundert Zyklen beim Mikrofluidizer).

Die Charakterisierung der hochfeinen Mikropartikeldispersionen und Nanopartikel erfolgte mit Laser Diffraktometrie (LD) (Coulter LS230, Firma Coulter Electronics, Miami, USA) und mit Photonenkorrelationsspektrosköpie (PCS) (Zetasizer 4, Malvern Instruments, Malvern, United Kingdom). Charakterisierungsparameter waren der LD-Durchmesser 50% (D50%), 90% (D90%) und 95% (D95%) der mit dem LD gemessenen. Die PCS (Meßbereich ca. 3nm - 3µm) ergibt den PCS-Durchmesser und als Maß für die Breite der Verteilung den Polydispersitätsindex (PI) im Bereich von 0,000 (= ideal monodispers) bis 0,500 (sehr breite Verteilung), oberhalb von 0,5 ist keine Aussage über die Breite der Verteilung mehr möglich.

Die Feinheit der hergestellten Dispersion richtet sich nach dem Verwendungszweck Die Zielgröße für Partikel aus Polymeren liegt oft im Bereich weniger Mikrometer. Beispiele sind Dispersionen aus Ethylcellulose zum Überziehen von Tabletten oder Kortikoidbeladene Polylactid-glycolidpartikel zur Aufnahme durch Makrophagen nach intraarticulärer Injektion (Zielgröße ca. 1-2 µm). Für schwerlösliche Arzneistoffe liegt die Zielgröße oft im Bereich ca. 1 µm bzw. im Nanometerbereich, z.B. Azodicarbonarnid. Durch entsprechende Wahl von Druck und Zyklenzahl läßt sich die Zielgröße im Produktionsprozeß kontrollieren.

### Beispiele

Beispiel 1 (nicht erfindungsgemäβ) Der Arzneistoff 1-[[2,7-bis(2,6-dimethyl-4-morpholinyl)-6-phenyl-4-pteridinyl]-(2-hydroxyethyl)-amino]-2-methyl-[cis[cis]]-propan-2-ol (1%) wurde in wasserfreiem Glycerol unter Zusatz von Tween 80 (0,5%) dispergiert und dann die erhaltene Prä-Dispersion in einem diskontinuierlichen Micron LAB40 hochdruckhomogenisiert (APV Deutschland GmbH, Lübeck, Germany). Produktionsparameter waren 2 Zyklen bei 150 bar, dann 2 Zyklen bei 500 bar und anschließend 6 Zyklen bei 1500 bar. Homogenisation erfolgte bei Raumtemperatur. Partikelgrößenanalytik,mit dem Laserdiffraktometer Coulter LS230 (Coulter Electronics, USA). Nach den 6 Zyklen mit 1500 bar betrug der D50% 1,7 µm, der D90% 4,5 µm und der D95% 5,4 µm.

### Beispiel 2

Zur Herstellung von Nanopartikeln wurde der Arzneistoff aus Beispiel 1 wie dort beschrieben homogenisiert, wobei jedoch 20 Zyklen bei 1500 bar gefahren wurden. Der mit Photonenkorrelationsspektroskopie bestimmte mittlere PCS-Durchmesser betrug 950 nm, der PI 0,513.

### Beispiel 3

Das synthetische Polymer Eudragit RS PO (Polyacrylsäure-trimethylamino-ethylester., Röhm GmbH, Darmstadt, Germany) wurde in 10% unter Zusatz von 1,5% Tween 80 in Propylenglycol dispergiert. Die Partikelgrößenbestimmung des mit Ultraschall dispergierten Pulvers ergab einen D50% von 79,7 µm und einen D95% von 185 µm. Homogenisation erfolgte analog Beispiel 1 im diskontinuierlichen Micron LAB40, Produktionsparameter waren 2 Zyklen bei 150 bar, 2 Zyklen bei 500 bar und anschließend 2 Zyklen bei 1500 bar

(Raumtemperatur). Der PCS-Durchmesser der Nanopartikeldispersion betrug 123 nm, der Polydispersitätsindex 0,185. In Übereinstimmung waren damit der LD-Durchmesser D50% von 139 nm und der D99% von 149 nm.

## Patentansprüche

1. Verfahren zur schonenden Herstellung von hochfeinen Mikro-und Nanopartikeln mit einer Partikelgröße, angegeben als mittlerer Durchmesser der Anzahlverteilung, kleiner als 1 µm, **dadurch gekennzeichnet, daß** ein Matrixmaterial in einem wasserfreien oder wasserreduzierten Dispersionsmedium, das weniger als 50 Gew.% Wasser enthält, dispergiert wird und bei niedrigen Temperaturen unter 90 °C, vorzugsweise 20 °C und insbesondere unterhalb des Gefrierpunktes von Wasser, in einem Kolben-Spalt-Homogenisator einem Hochdruckhomogenisationsprozeß unterworfen wird, der zu einer schonenden Partikelzerkleinerung führt unter Minimierung der Beeinträchtigung der chemischen Stabilität des homogenisierten Materials.

2. Verfahren nach Anspruch 1, **dadurch gekennzeichnet, daß** es sich bei dem homogenisierten Matrixmaterial um Arzneistoffe, insbesondere pharmazeutische Wirkstoffe oder Veterinärarzneistoffe, oder um Wirkstoffe und/oder Hilfsstoffe und/oder Zusatzstoffe für Kosmetika, Agrarprodukte, Nahrungsmittel und konservierende Produkte handelt.

3. Verfahren nach Anspruch 2, **dadurch gekennzeichnet, daß** es sich bei dem homogenisierten Matrixmaterial um die Arzneistoffe Ciclosporin, Azodicarbonamid, Paclitaxel, Prednisolon, Carbamazepin, Taxol, Morphin, Diclofenac, Ibuprofen, Phenobarbital oder Cromoglicin handelt.

4. Verfahren nach Anspruch 1, **dadurch gekennzeichnet, daß** es sich bei dem homogenisierten Matrixmaterial um synthetische, halbsynthetische oder natürliche Polymere, insbesondere natürliche Makromoleküle handelt.

5. Verfahren nach Anspruch 4, **dadurch gekennzeichnet, daß** es sich bei dem homogenisierten Matrixmaterial um synthetische Polymere, insbesondere um die Polymere Polylactid, Polyglycolid, Polylactid/-glycolid-Copolymer, Polyorthoester, Polyhydroxybutyrat (PHB), Polyhydroxyvaleriat (PHV), Polyhydroxybutyrat/valeriat-Copolymer, Polyacrylate, Polymethacrylate, Polyvinylderivate, Blockpolymere aus Polyethylenglycol und Polyestern, Polyhydroxybuttersäure, Polycyanoacrylate, Polycarbonate oder Polycaprolacton, handelt.

6. Verfahren nach Anspruch 4, **dadurch gekennzeichnet, daß** es sich bei dem homogenisierten Matrixmaterial um natürliche Makromoleküle, insbesondere Alginate, Albumin, bevorzugt Serumalbumin, Humanalbumin und bovines Albumin, Kollagen, Kasein, Fibrin, Tragant, Xanthane, Polysaccharide, insbesondere Chitin, Dex-trane oder Hyaluronsäure handelt.

7. Verfahren nach Anspruch 1, **dadurch gekennzeichnet, daß** es sich bei dem homogenisierten Matrixmaterial um mit Arznei-oder Wirkstoff beladenen Polymere oder natürliche Makromoleküle handelt.

8. Verfahren nach Anspruch 7, **dadurch gekennzeichnet, daß** es sich bei dem mit Arznei- oder Wirkstoff beladenem homogenisierten Matrixmaterial um die Polymere Polylactid, Polyglycolid, Polylactid/-glycolid-Copolymer, Polyorthoester, Polyhydroxybutyrat (PHB), Polyhydroxyvaleriat (PHV), Polyhydroxybutyrat/valeriat-Copolymer handelt.

9. Verfahren nach Anspruch 7, **dadurch gekennzeichnet, daß** es sich bei dem mit Arznei- oder Wirkstoff beladenem homogenisierten Matrixmaterial um natürliche Makromoleküle, insbesondere Alginate, Albumin, bevorzugt Serumalbuinin, Humanalbumin und bovines Albumin, Kollagen, Kasein, Fibrin, Bentonit, Tragant, Xanthane, Polysaccharide wie Chitin, Dextrane oder Hyaluronsäure handelt.

10. Verfahren nach einem der Ansprüche 1 bis 9, **dadurch gekennzeichnet daß** die zu zerkleinernden Materialien in einem nichtwäßrigen Dispersionsmedium dispergiert sind.

11. Verfahren nach Anspruch 10, **dadurch gekennzeichnet daß** die zu zerkleinernden Materialien in einem öligen Medium, insbesondere mittelkettigen Trigylceriden (MCT), Erdnußöl, Rizinisöl, Baumwollsamenöl, Distelöl, langkettigen Triglyceriden (LCT), insbesondere Soyaöl, Triacetin oder Isopropylmyristat, dispergiert sind.

12. Verfahren nach Anspruch 10, **dadurch gekennzeichnet daß** die zu zerkleinernden Materialien in flüssigen Kohlenwasserstoffen, insbesondere dünnflüssigem Paraffin, dickflüssigem Paraffin, Hexan oder Octan, dispergiert sind.

13. Verfahren nach Anspruch 10, **dadurch gekennzeichnet daß** die zu zerkleinernden Materialien in Polyethylenglykolen (PEG), insbesondere PEG 100 bis PEG 1000, wasserfreiem Glycerol, wasserfreien Alkoholen, insbesondere Methanol, Ethanol, 1-Propanol, Isopropanol, n-Butanol, 2-Butanol, Pentanol, Hexanol, Octanol, Decanol, Allylalkohol, Propargylalkohol, Ethanol, Isopropanol und Butanol, oder Propylenglykolen dispergiert sind.

14. Verfahren nach Anspruch 10, **dadurch gekennzeichnet daß** die zu zerkleinernden Materialien in Dimethylsulfoxid dispergiert sind.

15. Verfahren nach einem der Ansprüche 1 bis 9, **dadurch gekennzeichnet daß** die zu zerkleinernden Materialien in einem Dispersionsmedium dispergiert sind, daß einen geringen oder minimierten oder produkttechnisch wünschenswerten Anteil an Wasser enthält, der weniger als 50 Gew.% beträgt.

16. Verfahren nach dem Anspruch 15, **dadurch gekennzeichnet daß** die zu zerkleinernden Materialien in einem Dispersionsmedium dispergiert sind, daß weniger als 5 Gew.%, insbesondere weniger als 1 Gew.% an Wasser enthält.

17. Verfahren nach dem Anspruch 15, **dadurch gekennzeichnet daß** die zu zerkleinernden Materialien in einem Dispersionsmedium dispergiert sind, daß weniger als 10 Gew.% an Wasser enthält.

18. Verfahren nach dem Anspruch 15, **dadurch gekennzeichnet daß** die zu zerkleinernden Materialien in einem Dispersionsmedium dispergiert sind, daß Wasser enthält, in dem weitere Substanzen gelöst sind, insbesondere Polymere, bevorzugt bei Raumtemperatur feste Polyethylenglykole, bevorzugt PEG 6000, oder Cellulosederivate, insbesondere Hydroxypropylmethylcellulose (HPMC).

19. Verfahren nach einem der Ansprüche 15 bis 18, **dadurch gekennzeichnet daß** die zu zerkleinernden Materialien in einem Medium gemäß einem der Ansprüche 10 bis 14 dispergiert sind, dem ein Anteil an Wasser zugefügt wurde.

20. Verfahren nach einem der Ansprüche 1 bis 19, **dadurch gekennzeichnet, daß** die Prozeßtemperatur oberhalb 20°C list, zugsweise jedoch unter 50° C und insbesondere unter 30° C.

21. Verfahren nach einem der Ansprüche 1 bis 19, **dadurch gekennzeichnet, daß** die Prozeßtemperatur 20°C ist, vorzugsweise darunter, insbesondere bei ca. 4°C.

22. Verfahren nach einem der Ansprüche 1 bis 19, **dadurch gekennzeichnet, daß** die Prozeßtemperatur unterhalb des Gefrierpunktes des Wassers ist, vorzugsweise unter -20°C und insbesondere unter -50 °C.

23. Verfahren nach einem der Ansprüche 1 bis 22, **dadurch gekennzeichnet, daß** die Prozeßführung unter Ausschluß von Sauerstoff erfolgt, insbesondere unter Begasung mit inerten Gasen, bevorzugt Stickstoff oder Argon, oder unter Vakuum erfolgt.

24. Verfahren nach einem der Ansprüche 1 bis 23, **dadurch gekennzeichnet, daß** beim Prozeß eingesetzte Dispersionsmedien vor Gebrauch entgast werden.

## Claims

1. Process for the gentle preparation of superfine micro- and nanoparticles with a particle size, referred to as average diameter of the number distribution, of less than 1 µm, **characterized in that** a matrix material is dispersed in an anhydrous or water-reduced medium containing less than 50 wt.-% water, and is subjected to a high-pressure homogenizing process in a piston-gap homogenizer at low temperatures below 90°C, preferably 20°C, and in particular below the freezing point of water, which results in a gentle particle reduction with minimization of the impairment of the chemical stability of the homogenized material.

2. Process according to claim 1, **characterized in that** the homogenized matrix material is drugs, in particular pharmaceutical active ingredients or veterinary drugs, or active ingredients and/or auxiliaries and/or additives for cosmetics, agricultural products, foodstuffs and preservatives.

3. Process according to claim 2, **characterized in that** the homogenized matrix material is the drugs ciclosporin, azodicarbonamide, paclitaxel, prednisolone, carbamazepine, taxol, morphine, diclofenac, ibuprofen, phenobarbital or cromoglycin.

4. Process according to claim 1, **characterized in that** the homogenized matrix material is synthetic, semi-synthetic or natural polymers, in particular natural macromolecules.

5. Process according to claim 4, **characterized in that** the homogenized matrix material is synthetic polymers, in particular the polymers polylactide, polyglycolide, polylactide/-glycolide copolymer, polyorthoester, polyhydroxybutyrate (PHB), polyhydroxyvaleriate (PHV), polyhydroxybutyrate/-valeriate copolymer, polyacryates, polymethacrylates, polyvinyl derivatives, block polymers of polyethylene glycol and polyesters, polyhydroxybutyric acid, polycyanoacrylates, polycarbonates or polycaprolactone.

6. Process according to claim 4, **characterized in that** the homogenized matrix material is natural macromolecules, in particular alginates, albumin, preferably serum albumin, human albumin and bovine albumin, collagen, casein, fibrin, tragacanth, xanthans, polysaccharides, in particular chitin, dextrans or hyaluronic acid.

7. Process according to claim 1, **characterized in that** the homogenized matrix material is polymers or natural macromolecules charged with drugs or active ingredient.

8. Process according to claim 7, **characterized in that** the homogenized matrix material charged with drug or active ingredient is the polymers polylactide, polyglycolide, polylactide/-glycolide copolymer, polyorthoester, polyhydroxybutyrate (PHB), polyhydroxyvaleriate (PHV), polyhydroxybutyrate/-valeriate copolymer.

9. Process according to claim 7, **characterized in that** the homogenized matrix material charged with drug or active ingredient is natural macromolecules, in particular alginates, albumin, preferably serum albumin, human albumin and bovine albumin, collagen, casein, fibrin, bentonite, tragacanth, xanthans, polysaccharides such as chitin, dextrans or hyaluronic acid.

10. Process according to one of claims 1 to 9, **characterized in that** the materials to be reduced are dispersed in a non-aqueous/anhydrous dispersion medium.

11. Process according to claim 10, **characterized in that** the materials to be reduced are dispersed in an oily medium, in particular medium-chained triglycerides (MCT), peanut oil, ricinus oil, cottonseed oil, safflower oil, long-chained triglycerides (LCT), in particular soybean oil, triacetin or isopropyl myristate.

12. Process according to claim 10, **characterized in that** the materials to be reduced are dispersed in liquid hydrocarbons, in particular low viscosity paraffin, viscous paraffin, hexane or octane.

13. Process according to claim 10, **characterized in that** the materials to be reduced are dispersed in polyethylene glycols (PEGs), in particular PEG 100 to PEG 1000, anhydrous glycerol, anhydrous alcohols, in particular methanol, ethanol, 1-propanol, isopropanol, n-butanol, 2-butanol, pentanol, hexanol, octanol, decanol, allyl alcohol, propargyl alcohol, ethanol, isopropanol and butanol, or propylene glycols.

14. Process according to claim 10, **characterized in that** the materials to be reduced are dispersed in dimethyl sulfoxide.

15. Process according to one of claims 1 to 9, **characterized in that** the materials to be reduced are dispersed in a dispersion medium, that contains a small or minimized proportion or proportion desired, for product-related reasons, of water, which is below 50 wt.-%.

16. Process according to claim 15, **characterized in that** the materials to be reduced are dispersed in a dispersion medium which contains less than 5 wit.-%, in particular less than 1 wt.-%, water.

17. Process according to claim 15, **characterized in that** the materials to be reduced are dispersed in a dispersion medium which contains, less than 10 wt.-% water.

18. Process according to claim 15, **characterized in that** the materials to be reduced are dispersed in a dispersion medium which contains water in which further substances are dissolved, in particular polymers, preferably polyethylene glycols solid at room temperature, preferably PEG 6000, or cellulose derivatives, in particular hydroxypropyl methylcellulose (HPMC).

19. Process according to one of claims 15 to 18, **characterized in that** the materials to be reduced are dispersed in a medium according to one of claims 10 to 14, to which a proportion of water has been added.

20. Process according to one of claims 1 to 19, **characterized in that** the process temperature is above 20°C, but preferably below 50°C and in particular below 30°C.

21. Process according to one of claims 1 to 19, **characterized in that** the process temperature is 20°C, preferably below this, in particular approx. 4°C.

22. Process according to one of claims 1 to 19, **characterized in that** the process temperature is below the freezing point of water, preferably below -20°C and in particular below -50°C.

23. Process according to one of claims 1 to 22, **characterized in that** the process is carried out with the exclusion of oxygen, in particular with gassing with inert gases, preferably nitrogen or argon, or under a vacuum.

24. Process according to one of claims 1 to 23, **characterized in that** dispersion media used in the process are degassed before use.

## Revendications

1. Procédé pour la production économe de micro- et de nanoparticules ultra-fines présentant une taille particulaire, indiquée comme diamètre moyen de la distribution numérique, inférieure à 1 µm, **caractérisé en ce qu'**un matériau de matrice est dispersé dans un milieu de dispersion exempt d'eau ou réduit en eau, qui contient moins de 50 % en poids d'eau, et est soumis, à de basses températures inférieures à 90 °C, de préférence 20 °C et, en particulier, inférieures au point de congélation de l'eau, dans un homogénéisateur piston-fente, à un procédé d'homogénéisation à haute pression, qui conduit à un broyage des particules économe dans des conditions minimisant l'altération de la stabilité chimique du matériau homogénéisé.

2. Procédé selon la revendication 1, **caractérisé en ce qu'**il s'agit, dans le cas du matériau de matrice homogénéisé, de produits pharmaceutiques, en particulier de substances actives pharmaceutiques ou de produits pharmaceutiques vétérinaires ou de substances actives et/ou d'excipients et/ou d'additifs pour des produits cosmétiques, des produits agricoles, des produits alimentaires et des produits conservateurs.

3. Procédé selon la revendication 2, **caractérisé en ce qu'**il s'agit, dans le cas du matériau de matrice homogénéisé, de produits pharmaceutiques, tels que la ciclosporine, l'azodicarbonamide, le paclitaxel, la prednisolone, la carbamazépine, le taxol, la morphine, le diclofénac, l'ibuprofène, le phénobarbital ou la cromoglicine.

4. Procédé selon la revendication 1, **caractérisé en ce qu'**il s'agit, dans le cas du matériau de matrice homogénéisé, de polymères synthétiques, semi-synthétiques ou naturels, en particulier de macromolécules naturelles.

5. Procédé selon la revendication 4, **caractérisé en ce qu'**il s'agit, dans le cas du matériau de matrice homogénéisé, de polymères synthétiques, en particulier des polymères de type polylactide, polyglycolide, copolymère de polylactide/polyglycolide, poly(orthoester), polyhydroxybutyrate (PHB), polyhydroxyvalériate (PHV), copolymère de polyhydroxybutyrate/polyhydroxyvalériate, polyacrylate, polyméthacrylate, dérivés de polyvinyle, polymères séquencés de polyéthylèneglycol et de polyesters, poly(acide hydroxybutyrique), polycyanoacrylate, polycarbonate ou polycaprolactone.

6. Procédé selon la revendication 4, **caractérisé en ce qu'**il s'agit, dans le cas du matériau de matrice homogénéisé, de macromolécules naturelles, en particulier d'alginates, d'albumine, de préférence de sérumalbumine, d'albumine humaine et d'albumine bovine, de collagène, de caséine, de fibrine, d'adragante, de xanthane, de polysaccharides, en particulier de chitine, de dextrane ou d'acide hyaluronique.

7. Procédé selon la revendication 1, **caractérisé en ce qu'**il s'agit, dans le cas du matériau de matrice homogénéisé, de polymères chargés d'un produit pharmaceutique ou d'une substance active, ou de macromolécules naturelles.

8. Procédé selon la revendication 7, **caractérisé en ce qu'**il s'agit, dans le cas du matériau de matrice homogénéisé chargé d'un produit pharmaceutique ou d'une substance active, des polymères de type polylactide, polyglycolide, copolymère de polylactide/polyglycolide, poly(orthoester), polyhydroxybutyrate (PHB), polyhydroxyvalériate (PHV), copolymère de polyhydroxybutyrate/polyhydroxyvalériate.

9. Procédé selon la revendication 7, **caractérisé en ce qu'**il s'agit, dans le cas du matériau de matrice homogénéisé chargé d'un produit pharmaceutique ou d'une substance active, de macromolécules naturelles, en particulier d'alginates, d'albumine, de préférence de sérumalbumine, d'albumine humaine et d'albumine bovine, de collagène, de caséine, de fibrine, de bentonite, d'adragante, de xanthane, de polysaccharides tels que la chitine, le dextrane ou l'acide hyaluronique.

10. Procédé selon l'une quelconque des revendications 1 à 9, **caractérisé en ce que** les matériaux à broyer sont dispersés dans un milieu de dispersion non aqueux.

11. Procédé selon la revendication 10, **caractérisé en ce que** les matériaux à broyer sont dispersés dans un milieu huileux, en particulier des triglycérides à chaîne moyenne (MCT), de l'huile d'arachide, de l'huile de ricin, de l'huile de graines de coton, de l'huile de chardon, des triglycérides à chaîne longue (LCT), en particulier de l'huile de soja, de la triacétine ou du myristate d'isopropyle.

12. Procédé selon la revendication 10, **caractérisé en ce que** les matériaux à broyer sont dispersés dans des hydrocarbures liquides, en particulier de la paraffine liquide, de la paraffine visqueuse, de l'hexane ou de l'octane.

13. Procédé selon la revendication 10, **caractérisé en ce que** les matériaux à broyer sont dispersés dans des polyéthylèneglycols (PEG), en particulier ceux de la gamme PEG 100 à PEG 1000, du glycérol exempt d'eau, des alcools exempts d'eau, en particulier le méthanol, l'éthanol, le 1-propanol, l'isopropanol, le n-butanol, le 2-butanol, le pentanol, l'hexanol, l'octanol, le décanol, l'alcool d'allyle, l'alcool de propargyle, l'éthanol, l'isopropanol et le butanol, ou des propylèneglycols.

14. Procédé selon la revendication 10, **caractérisé en ce que** les matériaux à broyer sont dispersés dans du sulfoxyde de diméthyle.

15. Procédé selon l'une quelconque des revendications 1 à 9, **caractérisé en ce que** les matériaux à broyer sont dispersés dans un milieu de dispersion qui contient une proportion en eau restreinte ou minimisée ou techniquement souhaitable, qui est inférieure à 50 % en poids.

16. Procédé selon la revendication 15, **caractérisé en ce que** les matériaux à broyer sont dispersés dans un milieu de dispersion qui contient moins de 5 % en poids, en particulier moins de 1 % en poids d'eau.

17. Procédé selon la revendication 15, **caractérisé en ce que** les matériaux à broyer sont dispersés dans un milieu de dispersion qui contient moins de 10 % en poids d'eau.

18. Procédé selon la revendication 15, **caractérisé en ce que** les matériaux à broyer sont dispersés dans un milieu de dispersion qui contient de l'eau, dans laquelle d'autres substances sont dissoutes, en particulier des polymères, de préférence des polyéthylèneglycols solides à température ambiante, de préférence le PEG 6000, ou des dérivés de cellulose, en particulier l'hydroxypropylméthylcellulose (HPMC).

19. Procédé selon l'une quelconque des revendications 15 à 18, **caractérisé en ce que** les matériaux à broyer sont dispersés dans un milieu selon l'une quelconque des revendications 10 à 14, auquel une proportion d'eau a été ajoutée.

20. Procédé selon l'une quelconque des revendications 1 à 19, **caractérisé en ce que** la température de procédé est supérieure à 20 °C, mais de préférence inférieure à 50 °C et, en particulier, inférieure à 30 °C.

21. Procédé selon l'une quelconque des revendications 1 à 19, **caractérisé en ce que** la température de procédé est de 20 °C, de préférence inférieure à celle-ci, en particulier d'environ 4 °C.

22. Procédé selon l'une quelconque des revendications 1 à 19, **caractérisé en ce que** la température de procédé est inférieure au point de congélation de l'eau, de préférence inférieure à -20 °C et en particulier inférieure à -50°C.

23. Procédé selon l'une quelconque des revendications 1 à 22, **caractérisé en ce que** la conduite du procédé s'effectue en l'absence d'oxygène, en particulier en insufflant des gaz inertes, de préférence de l'azote ou de l'argon, ou sous vide.

24. Procédé selon l'une quelconque des revendications 1 à 23, **caractérisé en ce que** les milieux de dispersion utilisés lors du procédé sont dégazés avant usage.
